# EUROPEAN PATENT APPLICATION

(11) **EP 1 562 039 A1**
(43) Date of publication of application: **10.08.2005**
(21) Application number: 03758935.5
(22) Date of filing: 27.10.2003
(51) Int. Cl.: G01N 22/00

(54) **PROBE FOR PHYSICAL PROPERTIES MEASUREMENT**

(30) Priority: 30.10.2002 JP 2002316770
(71) Applicant: Nichirei Corporation, Tokyo 104-8402 (JP); Tokai University, Tokyo 151-0063 (JP)
(72) Inventor: INOUE, Toshifumi, Funabashi-shi, Chiba 273-0005 (JP); YAGIHARA, Shin, Hiratsuka-shi, Kanagawa 254-0914 (JP); SHINYASHIKI, Naoki, Ashigarakami-gun, Kanagawa 258-0001 (JP)
(74) Representative: Khan, Mohammed Saiful Azam
(86) International application number: PCT/JP2003/013703
(87) International publication number: WO 2004/040282

(57) **Abstract**

The present invention relates to a probe (31) used in a physical property measuring apparatus that measures a complex dielectric constant of a measured object to measure a physical property value of the measured object on the basis of the measured complex dielectric constant, the physical property value being represented by the water content, the probe having an internal electrode (311) and an external electrode (312). An end surface (313) is formed obliquely to an axial direction of the internal electrode (311) in order to accurately measure the complex dielectric constant regardless of whether or not the surface of the measured object has roughness and to set an appropriate electric length.

## Description

### Technical Field

The present invention relates to a physical property measuring probe. More specifically, the present invention relates to improvements in a probe used for a physical property measuring apparatus that measures the complex dielectric constant of a measured object to measure a physical property value for the measured object on the basis of the complex dielectric constant measured, the physical property value being represented by the water content.

### Background Art

There are conventional apparatuses that measure the complex dielectric constant of a measured object to measure a physical property value for the measured object on the basis of the complex dielectric constant measured, the physical property value being represented by the water content. One of such apparatuses utilizes time domain reflectometry (referred to as a TDR method below), as in the case of a physical property measuring apparatus disclosed in Japanese Patent No. 2740528.

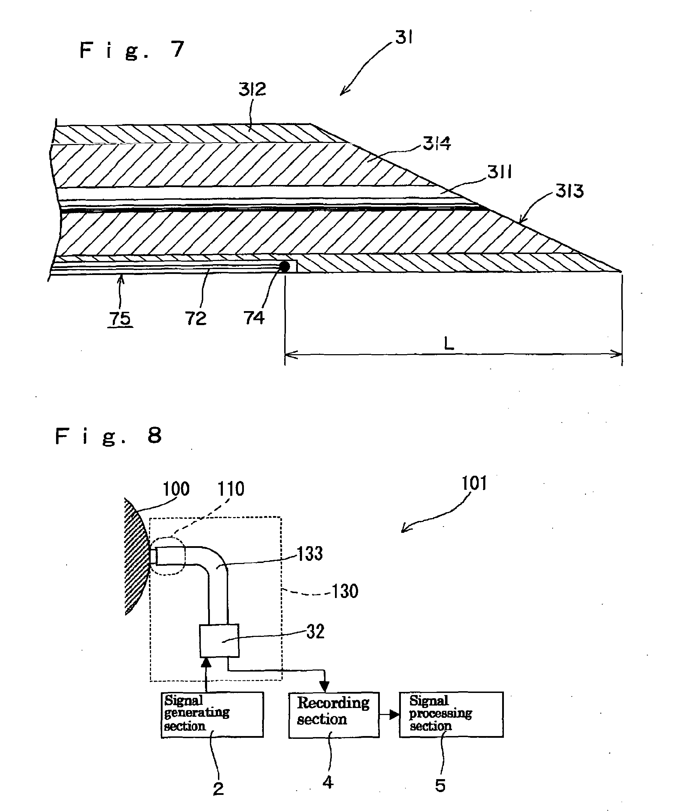

As shown in Fig. 8, this physical property measuring apparatus 101 comprises a signal generating section 2 that generates a step pulse provided to a measured object 100 and to a standard substance (not shown) whose complex dielectric constant is known, the step pulse serving as an excitation signal, a detecting section 130 which causes the excitation signal from the signal generating section 2 to enter the measured object 100 and the standard substance and which detects reflected waves from the measured object 100 and the standard substance, a recording section 4 that fetches and records, in temporal order, the reflected waves from the measured object 100 and the standard substance detected via the detecting section 130, and a signal processing section 5 that determines a difference between and the sum of the reflected waves from the standard substance and the measured object 100 recorded in the recording section 4, in terms of the frequency components of the reflected waves, the signal processing section 5 using the complex dielectric constant corresponding to a pre-recorded frequency component of the standard substance to determine the complex dielectric constant of the measured object 100. As the standard substance, a substance is selected the complex dielectric constant of which is close to that of the measured object 100. The standard substance is normally a liquid consisting of a single substance, for example, acetone, benzene, chloroform, or water.

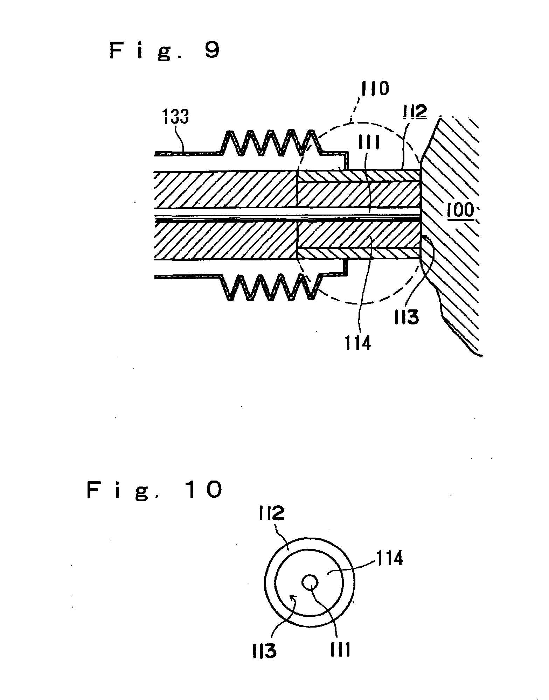

The detecting section 130 is composed of a probe 110 that comes in contact with the measured object 100, a sampling head 32 that acts as an interface between the signal generating section 2 and the recording section 4, and a coaxial cable 133 that connects the probe 110 to the sampling head 32. As shown in Figs. 9 and 10, the probe 110 has an internal conductor 111 that serves as a core wire, a cylindrical external conductor 112 placed coaxially with the internal conductor 111, and an insulator 114 placed between the internal conductor 111 and the external conductor 112. An end surface 113 that comes in contact with the measured object 100 is formed as a plane parallel with a cross section of the internal conductor 111, in other words, a plane perpendicular to an axial direction of the internal conductor 111.

The physical property measuring apparatus 101 allows a step pulse generated by the signal generating section 2 to enter the measured object 100, with the flat end surface 113 of the probe 110 being in contact with the measured object 100. The physical property measuring apparatus 101 then fetches and records a reflected wave from the measured object 100 in the recording section 4 in temporal order. Likewise, with the flat end surface 113 of the probe 110 in contact with the standard substance, the physical property measuring apparatus 101 allows a step pulse generated by the signal generating section 2 to enter the standard substance. The physical property measuring apparatus 101 then fetches and records a reflected wave from the standard substance in the recording section 4 in temporal order. Further, the measurement of the reflected wave may start with the measured object 100 or the standard substance. The signal processing section 5 determines a difference between and the sum of the reflected waves from the standard substance and the measured object 100. recorded in the recording section 4, in terms of the frequency components of the reflected waves. The signal processing section 5 then uses the complex dielectric constant corresponding to a pre-recorded frequency component of the standard substance to determine the complex dielectric constant of the measured object 100.

However, with the probe 110, when the measured object 100 has roughness on its surface, the measured object 100 cannot be contacted easily with the end surface 113. When the end surface 113 of the probe 110 is not in tight contact with the measured object 100, voids are involved in the measurement of the complex dielectric constant. Consequently, the complex dielectric constant of the measured object 100 cannot be accurately measured.

Further, if the measured object 100 is a food, the surface and inside of the food are expected to have different moisture conditions. Accordingly, the content of water in the food is desirably measured in order to accurately examine the freshness of the food. However, the probe 110 is of a type that makes its flat end surface 113 contact with parallel with the surface of the measured object 100. When the probe 110 is stuck into the measured object 100, pressing force acts uniformly on the flat end surface 113 in contact with the surface of the measured object 100. Consequently, it is difficult to stick the probe 110 into the measured object 100. Thus, the content of water in the measured object 100 cannot be measured easily using the probe 110. Further, when the probe 110 is forcibly thrust into the measured object 100, if the measured object 100 is a food containing particularly much moisture, the moisture may flow out of the measured object 100. As a result, the cell tissue of the measured object 100 may be destroyed.

In order to allow the probe 110 to be easily stuck into the measured object 100, it is possible to reduce the diameter of the probe 110 and thus the contact area between the probe 110 and the measured object 100. However, a reduction in the diameter of the probe 110 reduces the electric length of the electrode. Disadvantageously, a decrease in electric length may reduce the sensitivity of the probe 110.

Further, since the complex dielectric constant is affected by temperature, the temperature of the measured object 100 is measured when the complex dielectric constant is measured. Conventional techniques measure the complex dielectric constant and temperature using dedicated separate probes. Thus, if two physical quantities are simultaneously measured, the measurements must be made at different positions of the measured object 100. Consequently, the measurement points deviate from each other, that is, a positional deviation occurs. On the other hand, if the complex dielectric constant and temperature are measured at the same position of the measured object 100, one of the measurements must precede the other. Consequently, the measurement times deviate from each other, that is, a temporal deviation occurs. If the measured object 100 is placed in a heating or cooling process, for example, if the measured object 100 is a refrigerated food and this food is transferred to an area at room temperature for measurement, that is, if there is a large difference in temperature between the measured object 100 and the atmosphere, then the temperature may vary significantly among the parts of the measured object 100. Furthermore, the temperature may vary significantly over time. Therefore, with the conventional techniques that may cause the positional or temporal deviation, there may be a difference between the temperature at a position where the complex dielectric constant is measured or the temperature at a time when the complex dielectric constant is measured and the actually measured temperature. Thus, it is difficult to accurately measure the complex dielectric constant with respect to temperature.

### Disclosure of the Invention

It is an object of the present invention to provide a physical property measuring probe which can accurately measure the complex dielectric constant of a measured object regardless of whether or not the surface of the measured object has roughness and which can also measure a physical property value of the inside of the measured object, the probe also enabling the setting of an appropriate electric length. Further, it is another object of the present invention to provide a probe that can measure the temperature of vicinity of a measured position while simultaneously measuring the complex dielectric constant.

To accomplish this object, the present invention provides a physical property measuring probe used in a physical property measuring apparatus that measures the complex dielectric constant of a measured object to measure a physical property value of the measured object such as the water content on the basis of the measured complex dielectric constant, the probe having an internal electrode serving as a core wire and an external electrode placed coaxially with the internal electrode, wherein an end surface is formed obliquely to an axial direction of the internal electrode.

Accordingly, the end surface of the probe corresponds to a cross section obtained by obliquely cutting a cylinder. The end surface is thus elliptical. The length of the minor axis of this ellipse is the same as the outer diameter of the probe. The length of the major axis of the ellipse is determined by the angle of the end surface to the axial direction of the internal electrode. The length of the major axis is larger than the outer diameter of the probe. Consequently, the elliptical end surface has a larger area than a cross section of the probe. In this case, the electric length of the electrode increases consistently with increase in the area of the end surface of the probe. Therefore, the present invention makes it possible to increase the electric length without increasing the diameter of the whole probe. In other words, it is possible to set an appropriate electric length while reducing the diameter of the whole probe. Moreover, it is possible to adjust the length of the major axis of the elliptical end surface by adjusting the angle of the end surface to the axial direction of the internal electrode. Thus, the electric length can be adjusted in accordance with the type of the measured object or the like so that an appropriate sensitivity can be realized.

Moreover, according to the present invention, the end surface forms obliquely to the axial direction of the internal electrode. Consequently, the tip of the probe is sharp. When the probe is thrust into the measured object, pressing force concentrates on the sharp tip of the probe, which first comes in contact with the surface of the measured object. As a result, the probe can be easily stuck into the measured object. When the probe is stuck into the measured object, the end surface of the probe is made contact tightly with the measured object regardless of the shape of the surface of the measured object, that is, whether the surface has roughness. Consequently, the complex dielectric constant of the measured object can be accurately measured. Further, the probe has a sharp tip and may have a reduced diameter. This makes it possible to suppress damage to the measured object resulting from the thrust of the probe into the measured object. The probe according to the present invention can be easily manufactured by, for example, obliquely cutting a tip portion of a conventional semi-rigid cable.

Further, preferably, the physical property measuring probe according to the present invention is attached to a flexible probe-attached cable provided in the physical property measuring apparatus so that the probe can be freely attached and removed using coupling means. In this case, the flexible cable is deformed. This improves the degree of freedom in the operation of the probe. Further, when the probe can be freely attached to and removed from the cable, it can be easily replaced with another as required. For example, it is possible to replace the probe with one of a different electric length or of a type that is comes in contact with the surface of the measured object, as required.

Furthermore, a threaded structure is preferably used to attach the probe to the coupling means. In this case, the probe can be inexpensively configured so as to be freely attached to and removed from the cable. The orientation of the end surface of the probe can be adjusted by rotating or reversing only the probe in the direction of the thread without deformably twisting the cable.

Moreover, a temperature sensor is preferably placed near the end surface of the probe. In this case, it is possible to stick the probe into the measured object to measure its complex dielectric constant, while simultaneously measuring the temperature of vicinity of the measured point. Thus; even if the measured object is in a heating or cooling process, the complex dielectric constant can be accurately measured with respect to temperature.

### Brief Description of the Drawings

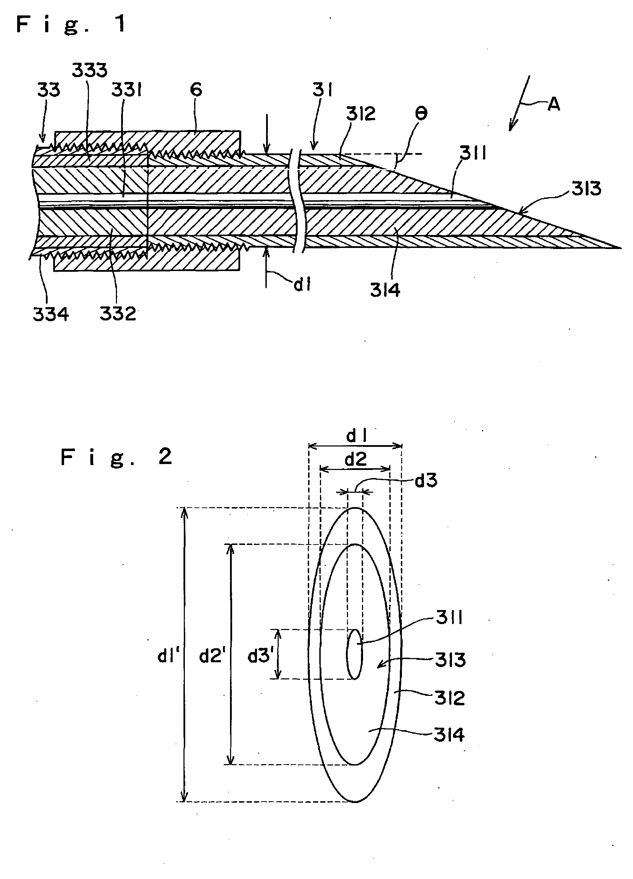

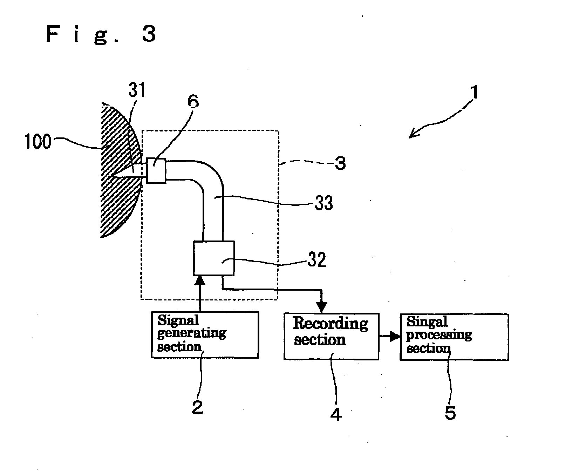

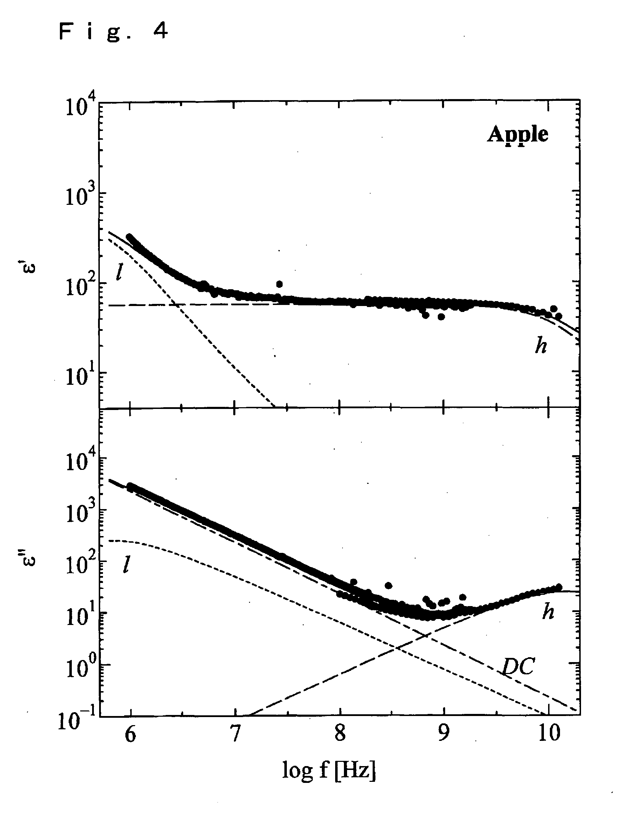

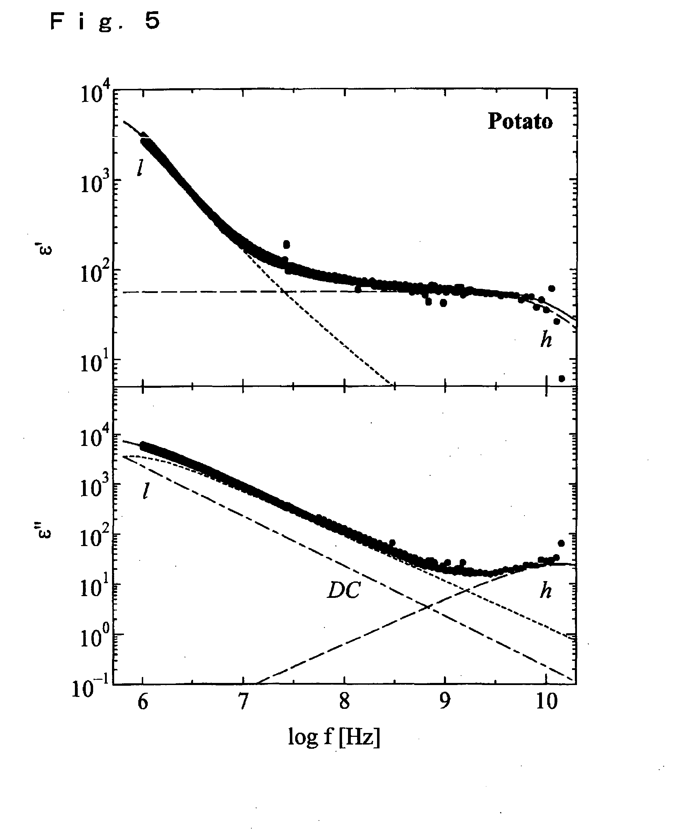

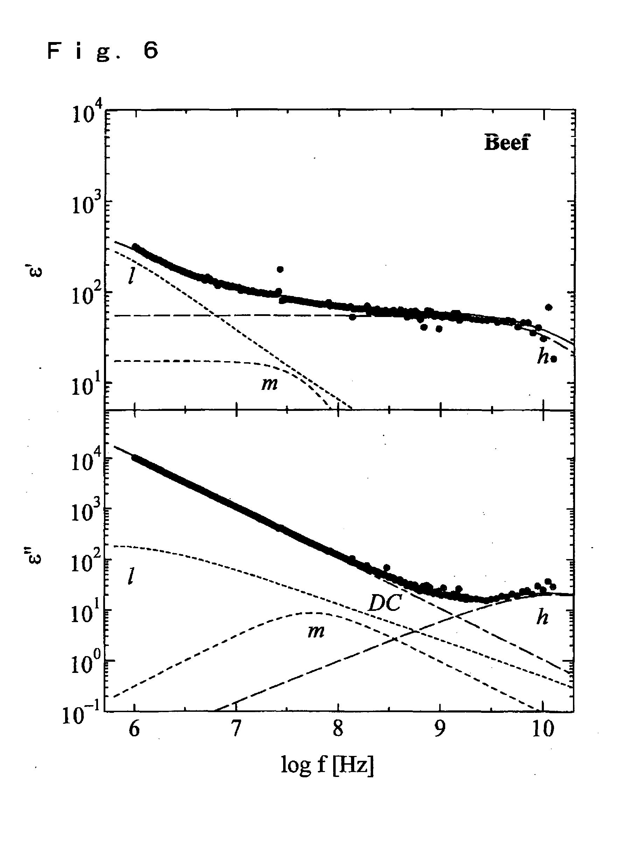

Fig. 1 is a central sectional view showing an embodiment of a physical property measuring probe according to the present invention. Fig. 2 is a diagram showing an end surface of the probe as viewed from the direction of arrow A in Fig. 1, which is perpendicular to the end surface of the probe. Fig. 3 is a block diagram showing an example of the configuration of a physical property measuring apparatus. Fig. 4 is a graph showing the results of measurements of the complex dielectric constant of an apple as a measured object which measurements were made using a TDR physical property measuring apparatus and an impedance analyzer both employing the physical property measuring probe according to the present invention, wherein the axis of abscissa indicates the logarithm log f Hz of a frequency, while the axis of ordinate indicates the real part ε' and imaginary part ε" of the complex dielectric constant. Fig. 5 is a graph showing the results of measurements of the complex dielectric constant of a potato as a measured object which measurements were made using the TDR physical property measuring apparatus and impedance analyzer both employing the physical property measuring probe according to the present invention, wherein the axis of abscissa indicates the logarithm log f Hz of the frequency, while the axis of ordinate indicates the real part ε' and imaginary part ε" of the complex dielectric constant. Fig. 6 is a graph showing the results of measurements of the complex dielectric constant of beef as a measured object which measurements were made using the TDR physical property measuring apparatus and impedance analyzer both employing the physical property measuring probe according to the present invention, wherein the axis of abscissa indicates the logarithm log f Hz of the frequency, while the axis of ordinate indicates the real part ε' and imaginary part ε" of the complex dielectric constant. Fig. 7 is a central sectional view showing another embodiment of a physical property measuring probe according to the present invention. Fig. 8 is a block diagram showing an example of the configuration of a conventional physical property measuring apparatus. Fig. 9 is a central sectional view showing a conventional physical property measuring probe. Fig. 10 is a front view showing the conventional physical property measuring probe.

### Best Mode for Carrying Out the Invention

The configuration of the present invention will be described below in detail on the basis of the best mode shown in the drawings.

Figs. 1 to 3 show an embodiment of a physical property measuring probe according to the present invention. This physical property measuring probe 31 is used in a physical property measuring apparatus 1 that measures the complex dielectric constant of a measured object 100 to measure a physical property value of the measured object 100 such as the water content on the basis of the measured complex dielectric constant.

The physical property measuring apparatus 1 employs, for example, the TDR method. As shown in Fig. 3, this physical property measuring apparatus 1 comprises a signal generating section 2 that generates a step pulse provided to a measured object 100 and to a standard substance (not shown) whose complex dielectric constant is known, the step pulse serving as an excitation signal, a detecting section 3 which causes the excitation signal from the signal generating section 2 to enter the measured object 100 and the standard substance and which detects reflected waves from the measured object 100 and the standard substance, a recording section 4 that fetches and records, in temporal order, the reflected waves from the measured object 100 and the standard substance detected via the detecting section 3, and a signal processing section 5 that determines a difference between and the sum of the reflected waves from the standard substance and the measured object 100 recorded in the recording section 4, in terms of the frequency components of the reflected waves, the signal processing section 5 using the complex dielectric constant corresponding to a pre-recorded frequency component of the standard substance to determine the complex dielectric constant of the measured object 100. The physical property measuring apparatus 1 has the same basic configuration as the conventional apparatus 101, shown in Fig. 8. The same components as those of the conventional apparatus 101 are denoted by the same reference numerals. Their detailed description is omitted.

The physical property measuring probe 31 has an internal electrode 311 that serves as a core wire and an external electrode 312 placed coaxially with the internal electrode 311. An end surface 313 is formed obliquely to an axial direction of the internal electrode 311. The internal electrode 311 and the external electrode 312 are composed of, for example, copper, which is a typical conductive material. An insulator 314 is placed between the internal electrode 311 and the external electrode 312. The material of the insulator 314 is, for example, polytetrafluoroethylene. The probe 31 can be easily obtained by, for example, obliquely cutting a tip portion of a conventional semi-rigid cable. To prevent the internal electrode 311 and the external electrode 312 from being corroded, it is preferable to plate, with gold or platinum, a metal portion of the end surface 313 and a peripheral surface of the external electrode 312, inserted into the measured object 100.

The following are set so that the probe 31 is thin and sharp and that an electric length γd is appropriate: the outer diameter d1 and inner diameter d2 of the external electrode 312, the diameter d3 of the internal electrode 311, and the angle θ of the end surface 313 to the axial direction of the internal electrode 311, all of which are shown in Fig. 2.

The electric length γd and the area of the end surface 313 are correlated. Specifically, the electric length γd increases consistently with increase in the area of the end surface 313, or decreases consistently with decreases in the area of the end surface 313. The end surface 313 of the probe 31 corresponds to a cross section of an obliquely cut cylinder. As shown in Fig. 2, the end surface 313 of the probe 31 is elliptical. The length of the minor axis of the ellipse is the same as the diameter of the probe 31 and as the length. of the outer diameter d1 of the external electrode 312 according to the present embodiment. The length of the major axis d1' of the ellipse is determined by the angle θ of the end surface 313 to the axial direction of the internal electrode 311. The length of the major axis d1' is expressed by d1' = d1 / sin θ and is equal to or larger than the diameter d1 of the probe 31. Consequently, the elliptical end surface 313 has a larger area than a cross section of the probe 31. The area of the end surface 313 increases consistently with increase in the major axis d1' of the ellipse. Consequently, by adjusting the angle θ of the end surface 313 to the axial direction of the internal electrode 311, it is possible to adjust the length of the major axis d1' of the elliptical end surface 313 and thus the area of the end surface 313, to obtain an appropriate electric length γd for the measured object 100. The probe 31 can be made more sensitive by increasing the electric length γd. Consequently, precise measurements can be made by selecting an appropriate electric length γd in accordance with the kind of the measured object 100 or the like.

Further, the probe 31 according to the present embodiment is electrically connected to a coaxial cable 33 provided in the physical property measuring apparatus 1 and serving as a probe-attached cable. The probe 31 is electrically connected to the signal generating section 2 and recording section 4. The coaxial cable 33 is preferably flexible. In this case, the coaxial cable 33 is deformed as the probe 31 moves. The direction or position of the probe 31 can thus be freely adjusted. The coaxial cable 33 has an internal conductor 331 that serves as a core wire, an insulator 332 that covers the internal conductor 331, an external conductor 333 that further covers the insulator 332, and an insulator 334 that further coats the external conductor 333. The internal electrode 311 of the probe 31 is electrically connected to the internal conductor 331 of the coaxial cable 33. The external electrode 312 of the probe 31 is electrically connected to the external conductor 333 of the coaxial cable 33. A sampling head 32 is connected to an end of the coaxial cable 33 which is opposite its end connected to the probe 31; the sampling head 32 serves as an interface between the signal generating section 2 and the recording section 4.

The probe 31 and the coaxial cable 33 are connected together using, for example, coupling means 6. The coupling means 6 according to the present embodiment has a threaded structure that mechanically connects the probe 31 and to the coaxial cable 33. Specifically, the coupling means 6 is formed as a nut that linearly couples the probe 31 and the coaxial cable 33. On the other hand, an external thread is formed at ends of the probe 31 and coaxial cable 33 screwed into the coupling means 6; the external thread engages with an internal thread of the coupling means 6. By employing the threaded structure for the coupling means 6, it is possible to remove the probe 31 and the coaxial cable 33 from the coupling means 6 as required. This allows the probe 31 to be configured so as to be freely attached to and removed from the coaxial cable 33. Further, by employing the threaded structure for the coupling means 6, it is advantageously possible to adjust the direction of the end surface 313 as desired by rotating or reversing only the probe 31 in the direction of the thread and without deformably twisting the coaxial cable 33.

In the above example, the coupling means 6 is attached to the coaxial cable 33 using the threaded structure. However, the coupling means 6 may be attached to the coaxial cable 33 so as not to be removed from it. In this case, the coupling means 6 may be fixed to the coaxial cable 33 by press-fitting, adhesion, soldering, or the like or may be attached to the coaxial cable 33 so as to be rotatable around the axis of the coaxial cable 33. The coupling means 6 is attached to the coaxial cable 33 so as to be rotatable around the axis of the coaxial cable 33, by, for example, forming a groove on the periphery of the coaxial cable 33, providing an annular projection on the inner periphery of a hole in the coupling means 6 into which the coaxial cable 33 is fitted, and fitting the annular projection into the peripheral groove of the coaxial cable 33. In this case, the probe 31 can be attached to the coaxial cable 33 by rotating only the coupling means 6 and without deformably twisting the coaxial cable 33 or rotating the probe 31 in the direction of the thread. Further, since the coupling means 6 rotates around the axis of the coaxial cable 33, the direction of the end surface 313 can be suitably adjusted as desired without deformably twisting the coaxial cable 33. However, the coupling means 6 is not limited to the one utilizing the threaded structure. Any means may be applied as required which electrically connects the probe 31 and the coaxial cable 33 together and which enables the probe 31 to be configured so as to be freely attached to and removed from the coaxial cable 33.

If the measured object 100 is a food and the amount of moisture contained in the food is measured using the physical property measuring apparatus 1, the following operation is performed. That is, the probe 31 is stuck into a food as the measured object 100. The end surface 313 of the probe 31 is made in contact tightly with the food regardless of whether or not the surface of the food has roughness. Consequently, the complex dielectric constant of the food can be accurately measured. Further, the probe 31 has a sharp tip and may have a reduced diameter. This makes it possible to suppress damage to the food as the measured object 100. The amount of moisture contained in the food can be determined on the basis of the measured complex dielectric constant of the food.

### <Example>

Now, description will be given of an example, that is, experiments made to confirm the effects of the present invention as well as their results. However, the example described below does not limit the present invention.

In the present experiments, for an apple, a potato, and beef as measured objects, the complex dielectric constant at room temperature was measured using a TDR physical property measuring apparatus comprising the probe 31 and an impedance analyzer comprising the probe 31. During the measurements, the temperature of each measured object was the same as the room temperature. The room temperature was about 26 to 27°C. Further, in the present embodiments, the electric length γd was 0.315 mm. The complex dielectric constant was measured by sticking the probe 31 into each measured object. In connection with conditions for measurements made by the TDR physical property measuring apparatus, the frequency range was set between 100 MHz and 10 GHz and air was used as a standard substance. In connection with conditions for measurements made by the impedance analyzer, the frequency range was set between 1 MHz and 1.8 GHz. Fig. 4 shows the results of measurements of the apple. Fig. 5 shows the results of measurements of the potato. Fig. 6 shows the results of measurements of the beef. In Figs. 4 to 6, the axis of abscissa indicates the logarithm of the frequency, that is, log f Hz, while the axis of ordinate indicates the real part ε' and imaginary part ε" of the complex dielectric constant.

Relaxation curves observed for the apple, the potato, and the beef were described by assuming two to three relaxation processes. For each measured object, one relaxation process associated with rotational diffusive motion of water molecules was observed on a high frequency side.

Further, one relaxation process mainly considered to result from electrode polarization was also observed on a low frequency side. Moreover, a dielectric loss associated with a DC conductive component was observed in the imaginary part of the complex dielectric constant. For the beef, the real part of the complex dielectric constant could not be described simply by the relaxation by the rotational diffusive motion of water molecules on the high frequency side and the relaxation by the electrode polarization on the low frequency side. Thus, one Debye type relaxation process was assumed for an intermediate frequency region between these relaxations. The relaxation process on the high frequency side is defined as h. The relaxation process on the low frequency side is defined as l. The relaxation process on the intermediate frequency region is defined as m.

Using these relaxation processes and a conductivity, the complex dielectric constant was described as shown below in Equation (1).
σ denotes the conductivity,
ε₀ denotes the dielectric constant on the low frequency side,
ε_{∞} denotes the dielectric constant on the high frequency side,
Δεᵢ denotes a relaxation intensity,
ω denotes an angular frequency,
τᵢ denotes a relaxation time, and
βᵢ denotes a parameter for the spread of the relaxation curve (βᵢ = 1; Debye type)

The above relaxation processes were subjected to curve fitting on the basis of Equation 1. Table 1 shows the relaxation parameters obtained. In the present embodiments, the dielectric constant ε_{∞} on the high frequency side was fixed to 5.0.

**Table 1**

| Measured object | τₛ [soc] | Δ_{δh} | βₕ | τᵢ [sec] | Δs₁ | βᵢ | σ [S·m⁻¹]· | τₘ [sec] | Δεₘ | βₘ |
|---|---|---|---|---|---|---|---|---|---|---|
| Apple | 1.08E-11 | 5.63E+01 | 0.90 | 2.38E-07 | 5.81E+02 | 091 | 1.25E-01 | - | - | - |
| Potato | 1.08E-11 | 5.63E+01 | 0.90 | 2.38E-07 | 8.43E+03 | 0.91 | 1.25E-01 | - | - | - |
| Beef | 1.15E-11 | 5.47E+01 | 0.81 | 2.73E-07 | 5.81E+02 | 0.72 | 5.88E-01 | 2.88E-09 | 1.72E+01 | 1.00 |

The above results of the experiments indicate that the complex dielectric constant of a solid sample can be measured by sticking the probe 31 into the solid sample, using a conventional TDR physical property measuring apparatus and impedance analyzer.

Moreover, the sticking type probe according to the present invention may be used to measure not only the complex dielectric constant but also another physical property value of a measured object at value other than the complex dielectric constant. Alternatively, the sticking type probe according to the present invention may also be provided with a section that detects a physical property value other than the complex dielectric constant.

For example, as shown in Fig. 7, a temperature sensor 72 is placed near the end surface 313. The temperature sensor is, for example, a thermocouple 72. However, the temperature sensor is not limited to the thermocouple but may be a well-known one employing a platinum resistance wire that is a temperature measuring resistor or thermister. If the thermocouple 72 is employed as a temperature sensor, a measuring contact 74 of the thermocouple 72 is placed near the end surface 313 of the probe 31. To attach the thermocouple 72 to the probe 31, it is possible to, for example, bond the thermocouple 72 to the outer periphery of the external electrode 312 using an adhesive or the like. Alternatively, a groove 75 may be formed in the external electrode 312 in its longitudinal direction to the extent that the groove 75 does not reach the insulator 314. The thermocouple 72 may then be fitted into the groove 75 and secured using an adhesive or the like. In the latter case, compared to the former case, the thermocouple 72 does not extend out from the external electrode 312, so that when the probe 31 is thrust into the measured object, the thermocouple 72 is advantageously unlikely to slip out of the probe 31. The relationship between the end surface 313 of the probe 31 and the thermocouple 72 is preferably set so that, for example, the probe 31 and the thermocouple 72 does not mutually disturb detection signals, that is, they do not disturb each other. To achieve this, it is preferable to, for example, place the measuring contact 74 of the thermocouple 72 slightly closer to the apparatus main body than the tip of the probe 31, as shown by L in Fig. 7. Means for measuring the complex dielectric constant may be, for example, a well-known apparatus employing the TDR method. The thermocouple 72 is a well-known one consisting of two different conductors the opposite ends of which are joined together. The thermocouple 72 is connected to temperature measuring means (not shown). The temperature measuring means is a well-known apparatus that utilizes a thermal electromotive force based on the Seebeck effect to measure a thermal voltage generated between two contacts of the thermocouple 72 and thus the temperature of the measuring contact 74. With the above configuration, by sticking the probe 31 into the measured object, it is possible to measure the complex dielectric constant of the measured object, while simultaneously measuring the temperature of vicinity of the measured point. Thus, even if the measured object is in a heating or cooling process, the complex dielectric, constant can be accurately measured with respect to temperature.

Moreover, by forming the probe such that its end surface forms obliquely, it is possible to adjust the area of the end surface to obtain a desired electric length. However, the desired electric length may be obtained on the basis of the cross section of the probe. The desired electric length may be obtained by, for example, forming the probe so that its end surface is a plane parallel with its cross section and that its cross section is shaped like an ellipse.

The above embodiment is a preferred embodiment of the present invention. However, the present invention is not limited to this. Many variations may be made to the above embodiment without departing from the gist of the present invention. For example, the measured object of the physical property measuring apparatus using the probe according to the present invention may be not only a food but also any general water-containing substance the moisture content of which must be determined. The physical property measuring apparatus using the probe according to the present invention is not limited to one employing the TDR method. In addition to the TDR method, for example, a frequency region measuring method is known as a method for measuring the complex dielectric constant of the measured object. The probe according to the present invention is applicable to a physical property measuring apparatus employing such a measuring method.

## Claims

1. A physical property measuring probe used in a physical property measuring apparatus that measures a complex dielectric constant of a measured object to measure a physical property value of the measured object such, as a water content, on the basis of the measured complex dielectric constant, the probe having an internal electrode serving as a core wire and an external electrode arranged coaxially with the internal electrode, the probe comprising forming an end surface thereof obliquely to an axial direction of the internal electrode.

2. The physical property measuring probe according to claim 1, wherein the probe is detachably attached to a flexible probe-attached cable provided in the physical property measuring apparatus through coupling means.

3. The physical property measuring probe according to claim 2, wherein the probe is attached to the coupling means by a threaded structure.

4. The physical property measuring probe according to claim 1, wherein a temperature sensor is arranged in the vicinity of the end surface.
